# EUROPEAN PATENT APPLICATION

(11) **EP 1 693 058 A1**
(43) Date of publication of application: **23.08.2006**
(21) Application number: 04719654.8
(22) Date of filing: 11.03.2004
(51) Int. Cl.: A61K 31/198, A61K 9/28, A61P 31/14, A61K 31/375

(54) **PREVENTION AND MEASURES FOR VIRAL INFECTION**

(30) Priority: 20.11.2003 JP 2003390695
(71) Applicant: Morishige, Fumie, Sanbu-gun, Chiba 299-3236 (JP); LIFE SCIENCE INSTITUTE LTD., Tokyo 1030012 (JP); CHOKO CO. LTD., Tokyo 1030012 (JP)
(72) Inventor: MORISHIGE, Fukumi, Chiba 2993236 (JP)
(74) Representative: Brady, Paul Andrew
(86) International application number: PCT/JP2004/003215
(87) International publication number: WO 2005/049007

(57) **Abstract**

The present invention relates to a method for preventing vial infection, which includes administering arginine and a high dose of vitamin C. It also relates to an agent for preventing vial infection, which includes arginine and coated vitamin C. According to the present invention, a subject can be efficaciously prevented from vial infection without the use of a vaccine. The present invention requires cost much lower than prevention methods using vaccines, might seldom invite adverse drug actions and do not induce severe adverse drug actions.

## Description

### Technical Field

The present invention relates to a method for preventing infection of various viruses, and a pharmaceutical preparation that can be used in the method.

### Background Art

Viruses are primitive biosystems that can grow only in living cells of animals. They have either one of DNA and RNA as their nucleic acid. This point distinguishes the viruses from bacteria. Examples of viral infectious diseases induced by viruses are those induced by DNA viruses, such as smallpox, herpes and hepatitis B; and those induced by RNA viruses, such as measles, influenza and Japanese encephalitis. Severe acute respiratory syndrome (SARS) is raging typically in China and is a viral infectious disease induced by coronavirus, a kind of RNA viruses.

Countermeasures to viral infectious diseases mainly comprise prevention using vaccines, since no antibiotics now used are efficacious to such viral infectious diseases. Vaccines are prepared by allowing a virus whose epidemic is anticipated to grow by incubation in hatching hen eggs, collecting allantoic cavity fluids containing a large amount of the virus, and inactivating or attenuating the virus typically by treatment with formalin. The resulting vaccine is not capable of infecting, namely not capable of growing in a host, but is capable of working as an antigen to allow the host to produce an antibody specific to the individual virus. The antibody traps and neutralizes an active virus. This mechanism allows the vaccine to prevent infection of the virus. The immunity raised by vaccination of the vaccine sustains for about one year, and the efficacy of vaccination is estimated to be 70% to 80%.

The prevention of viral infection by such a vaccine is, however, not always universal. Viruses always change their structures to yield new strains. A vaccine efficacious to a certain virus is not efficacious to a new strain of the virus which raised as a result of change in structure thereof. Among such viruses, RNA viruses severely change in their structures. There has been a report that coronavirus inducing SARS, for example, shows different nucleotide sequences between its strain in Hong Kong and that in Taiwan. Even if a vaccine efficacious for the prevention of SARS is developed, it may highly possibly become inefficacious immediately because of the rapid change in structure of the virus and is not promising.

The vaccination has various restrictions. The vaccination, for example, may invite adverse drug actions and must not applied to subjects with hypersensitivity to hen egg. When pandemy (nationwide or international epidemy over several countries) of swine influenzae was anticipated to occur in U.S.A. in 1976, the Federal Government prepared a vaccine with subsidy of as much as 16500 million dollars and inoculated the vaccine to millions of citizens. The feared epidemy, however, did not occur. Instead, severe adverse drug actions occurred in subjects subjected to vaccination, and the vaccination was aborted. The severest one among such adverse drug actions was Guillain-Barre syndrome which induces muscle weakness and severe sensory paralysis.

To inhibit viral infection without the use of vaccine, for example, a technique of administering, to a subject, a sufficient amount of serine leucocyte protease inhibitor, an analogue thereof or a derivative thereof to prevent infection is known (e.g., PCT Japanese Translation Patent Publication No. 8-505042). The publication says that this technique is efficacious for infectious diseases induced by retrovirus, such as cancer and autoimmune diseases, and can be preferably used as a countermeasure against infection of human immunodeficiency virus, such as acquired immune deficiency syndrome.

Accordingly, an object of the present invention is to solve the above-mentioned problems and to provide a novel method that can efficaciously prevent viral infection without the use of vaccines.

### Disclosure of Invention

After intensive investigations on the above object, the present inventors have found that viral infection can be effectively prevented by administering a high dose of vitamin C together with arginine to a subject. Accordingly, the present invention relates to a method for preventing viral infection, comprising administering arginine and a high dose of vitamin C.

The present invention also relates to an agent for preventing vial infection, comprising arginine and coated vitamin C.

### Best Mode for Carrying Out the Invention

Vitamin C (ascorbic acid) is a water-soluble vitamin and is involved in in-vivo redox reactions. Certain animals such as humans and monkeys or apes do not have an enzyme relating to the reaction of an intermediate of vitamin C and cannot biosynthesize vitamin C. A condition which is called typically as hypoascorbicacidemia or induced oligascorbicacidosis with a decreased blood vitamin C level generally induces disorder in the immune system, disorder in the blood coagulation system, incontinence to stress due to hypofunction of the adrenal gland, disorder in collagen production, malfunction in the nervous system, or onset of cancer caused by hypofunction of the immune or decreased detoxication capability against carcinogen. The present inventors have found infection of a virus, typically of coronavirus which induces SARS, can be prevented by administering a high dose of vitamin C in combination with arginine. The present invention has been accomplished based on these findings.

Vitamin C always undergoes autoxidation to release radicals. Copper, one of trace nutrients essential for living body, is chelated by the action of a large amount of vitamin C and becomes a metal complex to produce potent active oxygen. The potent active oxygen radical produced by the action of vitamin C and a trace amount of copper acts as an active substance in the method for preventing viral infection according to the present invention.

An action of vitamin C in the method for preventing viral infection of the present invention is to act upon and cleave the nucleotide sequence of a virus to thereby inactivate the virus. Such a virus injects its nucleotide sequence into a cell, parasitizes and grows therein. Upon the invasion into the cell, the nucleotide sequence escapes from its outer shell (envelope) and inner shell (protein layer) and becomes a naked DNA or RNA. The active oxygen radical attacks the naked nucleotide sequence to cleave part thereof. Upon the completion of parasitism, the virus escapes out of the cell in which the virus has parasitized to invade another cell. The DNA or RNA also becomes naked upon the escape and the resulting naked nucleotide sequence is attacked by the active oxygen radical. These attacks kill the virus to prevent the infection. Such radicals attack and cleave a single strand more easily than a double strand. It should therefore be understood that the prevention method of the present invention is more efficacious to RNA viruses.

Another action of vitamin C in the method for preventing vial infection of the present invention is to place a living body into a normal reduced condition from peroxidized condition. A virus invading the living body causes inflammation in various organs thereof to place the living body in a peroxidized condition. If the peroxidized condition is left stand, the infection of the virus advances, and the living body finally develops a vial infection. The vial infection, however, does not advance so far by returning the inflammation-induced peroxidized condition to a reduced condition. As a result, the vial infection can be prevented.

In addition, vitamin C acts to intensify the immune function, the defense mechanism which the human body inherently has. In this action, vitamin C can assist the function of leukocytes playing a predominant role in the immune function and thus enhance the immune function.

The dose of vitamin C is at least 300 to 500 mg/kg/day (20 g or more per day) for the prevention of virus, while varying depending on various conditions such as body weight, sexuality, age and health of the subject. If a living organ suffers from a disorder caused by viral infection, the disorder can be returned to the normal condition by administering vitamin C at least 1000 mg/kg/day. The recommended intake of vitamin C is generally considered to be about 1 mg/kg/day and to be up to 200 mg/day or less. It can be easily understood how high is the dose of vitamin C used in the prevention method of the present invention by comparing with these amounts. The dose herein is applied to oral administration.

In contrast, the upper limit of the dose of vitamin C is determined in accordance with the maximal permissible dose in the intestine of the subject. The "maximal permissible dose in the intestine of the subject" is the maximal amount at which vitamin C can be taken orally without inducing uncomfortable diarrhea, and is about 4 to 15 g per day in a normal human subject. This amount, however, varies from subject to subject and varies from time to time even in the same subject. The maximal permissible dose has been reported to be very high in a subject suffering from a severe disorder and decrease with recovery thereof. The maximal permissible dose may exceed 200 g/day in some subjects suffering from a severe disorder.

Points to note in the administration of vitamin C are as follows.
1) For oral administration, it is convenient to dissolve the necessary amount of vitamin C in about 2 liters of a beverage such as a juice and drink it in about ten installments. Vitamin C is preferably dissolved each time immediately before administration, because 50% of vitamin C once dissolved in a juice becomes an oxidized derivative within about 90 minutes and its effects decrease.
2) Vitamin C has a diuretic effect, but frequent urination presents no problem.
3) There was the theory that administration of vitamin C at a high dose induces ureteral stones, but it is now denied. No calcium precipitation causing calculus is induced by the administration of vitamin C, although aciduria is induced.
4) In the administration of vitamin C by drip infusion, pH of the drip infusion must be controlled at around 6.5. The activity of vitamin C decreases if the drip infusion has a pH of 7.0 or greater.
5) Vitamin C always undergoes autoxidation. The autoxidation of vitamin C has been conventionally prevented using an amino acid (cystine or cysteine) or glutathione. However, a high dose of cystine and cysteine invites imbalance in amino acids and is unsuitable for inhibiting the autoxidation of vitamin C in a high dose.
6) A high dose of vitamin C may invite chelating of in vivo mineral components to induce, for example, a hypokalemic, hypomagnesemic and/or hypocalcemic condition, and such conditions should be avoided. A dose of about 50 g/day, however, does not induce this phenomenon.
7) Tests of administering ¹⁴C (isotope)-labeled vitamin C to animals show that 70% of ¹⁴C is excreted into the expiration as ¹⁴CO₂ and residual 20% thereof is excreted into the urine. The general opinion that all of a high dose of vitamin C is immediately excreted into the urine must be reconsidered.

Arginine is administered together with a high dose of vitamin C according to the prevention method of the present invention. Arginine is one of twenty and several types of alpha-amino acids present in living body and is a basic amino acid. There are eight essential amino acids which the human cannot synthesize in vivo and must take as food. Arginine is not included in these essential amino acids but is classified as a semi-essential amino acid, because it is essential for the growth in childhood, although it can be synthesized in vivo in adult. Recently, however, the nutritional effect of arginine has been reassessed, showing that a deficiency of arginine causes metabolic disorders also in mature animals.

An action of arginine in the method for preventing vial infection of the present invention is to reinforce and assist the action of vitamin C. In particular, the administered arginine enhances the immune function. The immune system cells playing a role in the immune function include neutrophils and macrophages which are independent on antibodies, and lymphoid cells which are dependent on antibodies. The administration of arginine enhances or increases the functions of these. This has been verified, for example, from the following facts. Arginine administered to rats increases the weight of the thymus gland and T-cells' capability of blast transformation. When a rat suffers from fracture and the resulting decreased weight of the thymus gland and decreased T-cells' capability of blast transformation, it is recovered by administering arginine. Rats suffering from artificial peritonitis show a remarkably increased survivorship by administering arginine.

Arginine enhances the immune function by the action of Noₓ (nitrogen oxides) derived from arginine. More specifically, arginine administered to a subject produces Noₓ. The NOₓ acts to enlarge the blood vessel to thereby improve the circulation in organs.

The dose of arginine is generally 3 g/day or more, while varying depending on various conditions such as body weight, sexuality, age and health of the subject. If a desired effect is not obtained at a dose of 3 g/day, the dose can be increased, for example, to 6 g/day, and further to 9 g/day.

In contrast, an excessively high dose of arginine may induce excessive NOₓ to invite adverse effects. Excessive NOₓ, for example, is bound to hemoglobin in the red blood cell to form methemoglobin. Methemoglobin inhibits the binding between oxygen and hemoglobin to induce a decreased partial pressure of oxygen in the blood. Methemoglobin is known to bind with a secondary amine in vivo to form nitrosamine, a potent carcinogen. Such an excessively high dose of arginine, however, can be easily prevented by observing the NOₓ level in the urine, because excessive NOₓ is excreted into the urine. The NOₓ level in the urine can be easily observed by assaying NO₂ and NO₃ or by using a tes-tape. In other words, arginine can be administered at an initial dose of 3 g/day, and if the desired effect is not obtained, at an increasing dose while observing the NOₓ level in the urine. If the NOₓ level excessively increases, the dose is decreased. The dose herein is applied to oral administration. In this connection, the blood NOₓ level does not work as an indicator of the dose, because arginine is rapidly pooled in the cerebral nerve system and muscle.

Arginine is administered together with vitamin C in the method for preventing vial infection of the present invention. Thus, the NO radicals formed by the administration of excessive arginine is scavenged by vitamin C. The combination use of vitamin C and arginine is also preferred from this point of view.

Coadministering arginine and vitamin C in the prevention of vial infection according to the present invention allows fibrosed collagen to regenerate into absorbable (soluble) collagen. This may resolve fibrogenesis in the connective tissue. Viral pneumonia, a kind of viral infectious diseases, always invites inflammation and abnormal proliferation (proliferation of acidic mucopolysaccharides and collagen) in the pulmonary interstitium. Coadministration of vitamin C and arginine allows vitamin C to cleave carbohydrate chains of the acidic mucopolysaccharides and soften collagen. Thus, pulmonary insufficiency caused by viral pneumonia which has become chronic can also be prevented. The cleavage of hydrocarbon chains of acidic mucopolysaccharides by vitamin C can be verified in the following manner. When vitamin C is added to mucopolysaccharides and the viscosity of the mixture is observed with an Oswald viscometer, the viscosity gradually decreases.

Vitamin C and arginine must be administered for the prevention of vial infection according to the present invention. If the two ingredients are mixed before administration, the mixture undergoes Maillard reaction and becomes brown. The Maillard reaction once occurred inhibits the effects of the individual components and deteriorates the taste of the preparation. Accordingly, an agent for preventing vial infection comprises arginine and coated vitamin C and is preferably used in the method for preventing vial infection of the present invention. Vitamin C in the pharmaceutical preparation is coated with a coating, is not in direct contact with arginine and is free from the Maillard reaction. The coated vitamin C may be vitamin C fully coated with an oil or fat. The formulation of the agent for preventing vial infection is, for example, one comprising 10 g of vitamin C, 3 g of arginine and 1.5 g of RNA.

The prevention of vial infection according to the present invention can also employ a liquid suitable for drip infusion or injection. The liquid pharmaceutical preparation is efficacious when the subject is unconscious due to viral infectious disease or is an elderly and cannot take the pharmaceutical preparation orally. Upon such a formulation of a liquid pharmaceutical preparation, the Maillard reaction between the amino acid and a saccharide must be avoided. The Maillard reaction once occurred inhibits the effects of the individual components and deteriorates the taste of the preparation. The present inventors, however, have found that a mixture of L-arginine and L-ascorbic acid as an aqueous solution does not invite the Maillard reaction over several months, if it does not contain cystine and cysteine. They also have found that the duration until the Maillard reaction occurs can be prolonged by adding a sugar alcohol such as xylitol or sorbitol thereto.

Accordingly, the present invention also relates to an agent for preventing vial infection which comprises vitamin C and arginine dissolved in a pharmaceutically acceptable solution and contains no cysteine.

The present invention can prevent infection of a wide variety of viruses. Examples of such viruses are adeno-associated, batai, Bunyanwera, California encephalitis, coronavirus, cow-pox, Coxsackie (all the types A and B), Creutzfeldt-Jacob factor, dengue (all types), echovirus (all types), eastern equine encephalitis, enterovirus (68 to 71), EB, hepatitis (A, B, C, D and E), herpes simplex (1 and 2), human cytomegalovirus, HHV6, human papillomavirus, human parvovirus, human rhinovirus, human rotavirus, HTLV-I, HTLV-II, influenza (A, B and C), Japanese encephalitis, JC, lacrosse, LCM, measles, mumps, Murray Valley encephalitis, monkeypox, Newcastle disease, parainfluenza, poliovirus, rabies (fixed virus), RS, rubella, Semliki forest fever, simian immunodeficiency (SIV), sindbis, Saint Louis encephalitis, tanapox, vaccinia, varicella-zoster, vesicular stomatitis, western equine encephalomyelitis, West Nile fever, and Yaba monkey tumour pox viruses. The method for preventing vial infection of the present invention is efficacious for viral infection caused by either of DNA viruses and RNA viruses. The method is typically preferably used against coronavirus which causes severe acute respiratory syndrome (SARS).

The present invention will be illustrated in further detail with reference to several examples below, but these embodiments are not intended to limit the scope of the invention.

### EXAMPLE 1: Male in his seventies

A 70-year-old male suffering from influenzal pneumonia was a subject. Butter-fly-like shadows were observed in the bilateral hilar regions in the chest roentgenogram of the subject, showing that he suffered from pneumoniae. No antibiotic administration to the subject had been efficacious.

To the subject, 1000 mg/kg/day of vitamin C and 3 g/day of arginine were administered.

After 4-week administration, the subject recovered to be capable of walking, and his partial pressure of oxygen in the blood was returned to a normal level.

### EXAMPLE 2: Male in his seventies

A male in his seventies with decreased pulmonary function was a subject. The subject required oxygen inhalation continuously. The inspection of the upper body showed depression of the superior fovea of cheek bone upon inspiration. The respiratory sound was week in the auscultation, and the prolongation of the expiratory phase and expiratory time was observed. The percussion on the chest showed tympanitic resonance, indicating over-expansion of the lung. The chest CT finding of the subject showed alveolar ectasia. Such alveolar ectasia is caused by laceration of pulmonary interstitial fibers and is a symptom of pulmonary emphysema. The blood gas analysis showed a decreased partial pressure of oxygen in the blood, indicating venous admixture in the lung, i.e., the mixture of blood which is not involved in oxygen gas exchange in the lung with blood in the arterial system.

To the subject, 1000 mg/kg/day of vitamin C and 3 g/day of arginine were administered. Only a trivial effect was observed at this dose of arginine. Thus, the dose of arginine was gradually increased while observing the NOₓ level in the urine to avoid excretion of excessive NOₓ. A sufficient effect was obtained at a dose of 6 g/day. If the sufficient effect is not obtained at such a dose, the dose of arginine can be further increased, for example, to 9 g/day, as long as excessive NOₓ is not excreted into the urine.

After one-month administration, oxygen inhalation became unnecessary, the partial pressure of oxygen and the partial pressure of carbon dioxide in the blood stood at 85 or more and about 40, respectively, approaching normal levels. The NOₓ level in the urine stood at a normal level in spite of the regular use of arginine.

### EXAMPLE 3

Five parts by weight of arginine was added per 1 part by weight of vitamin C to a vitamin C injection containing no cysteine, a product of Fuso Pharmaceutical Industries, Ltd., to give a pharmaceutical preparation in the form of a solution.

The pharmaceutical preparation was administered to a patient with influenzal pneumonia by drip infusion. No antibiotic administration to the patient had been efficacious. The dose was set at such a level that the NOₓ level did not increase in the measurement of the NOₓ level in the urine of the patient in the measurement using a tes-tape.

The administration allowed the subject to recover immediately without showing adverse drug actions caused by a high dose of arginine.

### Industrial Applicability

According to the present invention, subjects such as animals including human beings whose viral infection should be prevent can be effectively prevent by administering arginine and a high dose of vitamin C. In addition, the cost for the prevention method of the present invention is much lower than that in prevention methods using vaccines, because vitamin C can be prepared in mass production at low cost, and arginine is contained in protamine, a nucleoprotein of sperms of fishes, in a large amount. The administration of vitamin C and arginine might seldom invite adverse drug actions and may not induce severe adverse drug actions, because their permissible doses can be easily determined.

The present invention is particularly efficacious against RNA viruses. Viruses are classified as a DNA virus and an RNA virus. Efficacious vaccines can be produced against the former, because the former does not easily change in its nucleotide sequence. However, no efficacious vaccine can be produced against the latter, because the latter easily changes its nucleotide sequence. The present invention, however, can surprisingly satisfactorily prevent the infection of an RNA virus, because the present invention directly acts upon the nucleotide sequence of the virus to kill the virus and is independent on the easiness of the change in nucleotide sequence.

## Claims

1. A method for preventing infection of a virus, comprising administering arginine and a high dose of vitamin C.

2. The method for preventing infection of a virus according to claim 1, wherein the dose of vitamin C is at least 300 to 500 mg/kg/day.

3. The method for preventing infection of a virus according to claim 2, wherein the dose of arginine is at least 3 g/day.

4. The method for preventing infection of a virus according to claim 1, wherein the administration is carried out orally.

5. The method for preventing infection of a virus according to claim 1, wherein the virus is coronavirus inducing SARS.

6. An agent for preventing infection of a virus, comprising arginine and coated vitamin C.

7. An agent for preventing infection of a virus, comprising vitamin C and arginine dissolved in a pharmaceutically acceptable solution and being free from cysteine.

8. Use of arginine and coated vitamin C as active ingredients for the production of an agent for preventing infection of a virus.
